## Europäisches Patentamt

## European Patent Office

## Office européen des brevets

(11) Veröffentlichungsnummer: **0 342 236**
**A1**

(12) # EUROPÄISCHE PATENTANMELDUNG

veröffentlicht nach Art. 158 Abs. 3 EPÜ

(21) Anmeldenummer: 88902615.9

(22) Anmeldetag: 20.11.87

Daten der zugrundeliegenden internationalen Anmeldung:

(86) Internationale Anmeldenummer: PCT/SU 87/00132

(87) Internationale Veröffentlichungsnummer: WO 89/04635 (01.06.89 89/12)

(51) Int. Cl.⁴: **A 61 B 8/08**, G 01 B 17/02

(43) Veröffentlichungstag der Anmeldung: 23.11.89 Patentblatt 89/47

(84) Benannte Vertragsstaaten: DE FR GB NL SE

(71) Anmelder: ESTONSKAYA SELSKOKHOZYAISTVENNAYA AKADEMIA, ul. Riia, 12, Tartu, 202400 (SU)
Anmelder: PYARNUSKAYA MEZHKOLKHOZNAYA SVINOFERMA, Pyarnusky raion, seisovet Sauga, 203600 (SU)

(72) Erfinder: KUUS, Mati Jukhanovich, ul. Purde, 4-6, Tartu, 202400 (SU)
Erfinder: YARV, Kalju Khansovich, ul. Side, 10a-6, Pyarnu, 203600 (SU)
Erfinder: MEISNER, Edvard Iokhannesovich, ul. Lauristini, 15-2, Tartu, 202400 (SU)
Erfinder: KAPP, Leo Peeterovich, ul. Oru, 2 Tartusky raion, p/o Peedu, 202443 (SU)

(74) Vertreter: Nix, Frank Arnold, Dr., Kröckelbergstrasse 15, D-6200 Wiesbaden (DE)

(54) **PULSECHO-PRÜFGERÄT.**

(57) Der Echoimpulstester weist einen Synchronisator (5) auf, dessen einer Ausgang mit einem der Eingänge einer Anzeigeeinheit (9) und dessen zweiter Ausgang mit dem Eingang eines Impulserzeugers (1) verbunden ist. Der Ausgang des Impulserzeugers (1) liegt am Eingang eines Verstärkers (4) und an einem der Eingänge eines Filters (2), an dessen anderen Eingang ein Ultraschallwandler (3) und an dessen Ausgang ein elektronischer Schalter (6) angeschlossen ist, der mit dem Steuereingang eines Speiseteils (8) in Verbindung steht. Der Ausgang des Speiseteils (8) liegt an den jeweiligen Eingängen des Impulserzeugers (1), des Synchronisators (5), des Verstärkers (4) und der Anzeigeeinheit (9).

Der Tester ist ebenfalls mit einem Echoimpulsdekadenzähler (7) und einer Trächtigkeitsdiagnoseschaltung (10) versehen, wobei der Ausgang des Verstärkers (4) an die entsprechenden Eingänge der Trächtigkeitsdiagnoseschaltung (10) und des Echoimpulszählers (7) angeschlossen ist, dessen Ausgänge an den Informationseingängen der Anzeigeeinheit (9) liegen. Der Ausgang der Anzeigeeinheit (9) ist an den anderen Eingang der Trächtigkeitsdiagnoseschaltung (10) angeschlossen, deren Ausgang am anderen Eingang des Verstärkers (4) liegt, und der andere Ausgang des Synchronisators (5) ist mit einem der Eingänge des Echoimpulszählers (7) verbunden, während der Ausgang des Speiseteils (8) an den jeweiligen Eingängen des Echoimpulszählers (7) und der Trächtigkeitsdiagnoseschaltung (10) liegt.

# ECHOIMPULSTESTER

## Gebiet der Technik

Die Erfindung bezieht sich auf die Ultraschallmesstechnik und betrifft insbesondere einen Echoimpulstester.

Die vorliegende Erfindung kann in der Tierzucht, hauptsächlich in der Schweinezucht zur Auslese (Selektion) der Schweine nach Erbmerkmalen (Fettschicht- und Rückenmuskeldicke) und zur Kontrolle der Frühgravidität (Trächtigkeit) nach ihrer Paarung oder künstlichen Insemination am wirksamsten ausgenutzt werden.

## Stand der Technik

In der Versorgung der Bevölkerung mit Nahrungsmitteln spielt die Schweinezucht eine wesentliche Rolle. Gleichzeitig steigt der Bedarf an qualitätshöherem, d.h. fettarmem Schweinefleisch. Zur Verminderung der Speckdicke werden die Schweine ständig nach erblichen Merkmalen ausgelesen. Die Dicke der Fett- und Muskelschicht zeichnet sich durch eine hohe Erblichkeitsziffer aus. Die Beurteilung von Fleisch-Fettqualitäten der Schweine ist daher für die richtige Auslese- und Zuchtorganisation in der Schweinehaltung von sehr grosser Bedeutung.

Für die Steigerung der Schweinefleischerzeugung und die Verbesserung der wirtschaftlichen Kennzahlen ist die bessere Sauenausnutzung von grossem Belang. Die Trächtigkeitsdiagnostik der Muttersauen dreissig Tage nach der Besamung zum Schlachtausmerzen jener Muttersauen, die sich als unbefruchtet erwiesen haben, ist daher von grosser Wichtigkeit.

Gegenwärtig ist der Bedarf an einem universalen kompakten Gerät zur Dickenmessung der Fett- und Muskelgewebeschicht sowie zur Trächtigkeitsfeststellung unmittelbar in Buchten aufgekommen, um die Arbeitsproduktivität der Viehpfleger in der Schweinezucht zu steigern und ihnen eine Arbeitserleichterung zu verschaffen sowie die Selbstkosten des Schweinefleisches zu senken.

Bekannt ist ein mechanisches Verfahren zur Dickenmessung der Fettschicht des Schweinefleisches durch Durchstechen (Durchstossen) der Tierhaut mit einem Messbolzen. Wegen der Reizung des Tieres und der Möglichkeit, es zu infizieren, ist dieses Verfahren nicht wirkungsreich.

- 2 -

Bekannt ist ein anderes Verfahren, bei dem Ultraschallwellen dem Tier zugeführt und dessen verschiedene Fettschichten durch Messung reflektierter Echoimpulse gemessen
werden. Da die Tiere unterschiedliche Gewebe- und Fettschichten haben, ruft jedoch jeder dem Tier zugeführte
Ultraschallimpuls eine Menge von den Trennschichten zwischen diesen Schichten reflektierter Echoimpulse hervor.

Das Problem besteht also darin, die erforderlichen
Echoimpulse gegen die unerwünschten zu isolieren und eine
Ausgangsanzeige zu bewirken, die ein Messergebnis in
leicht ablesbarer Form zu reproduzieren vermag, ohne dass
es vom Operateur verarbeitet werden muss. Bei den bekannten
Einrichtungen wurden Oszillographen zur Reflexionsimpulsanzeige verwendet, die den Nachteil hatten, dass die Messergebnisse von der Bedienungsperson verarbeitet werden
müssen.

Bekannt ist ein automatischer digitaler Dickenmesser
für die Fettschicht der Tiere (US, A, 4 359 056), der eine
Ultraschallimpulsübertragungs- und Reflexionsechosignalempfangsanordnung enthält, die einen am Körper des geprüften Tieres angeordneten Wandler aufweist. Der Wandler ist
an einen zur Verstärkung der vom Wandler empfangenen
Echosignale bestimmten Verstärker angeschlossen. Mit dem
Verstärker ist ein Zähler zum Abzählen der verstärkten
Echosignale verbunden, deren Amplitude ein vorgegebenes
Schwellenniveau überschreitet. Das Messgerät enthält eine
Vorrichtung zur Empfindlichkeitssteigerung beim Empfang
einer ersten vorgegebenen Anzahl der im Zähler abgezählten
Echosignale und zur Konstanthaltung der Apparateempfindlichkeit, nachdem diese Impulszahl abgezählt worden ist.
Zur Messung der Durchlaufzeit der Impulse im Tierkörper
ist eine Anzeigeeinheit vorgesehen. Sie tritt nach dem
Empfang einer zweiten vorgegebenen Anzahl der Echosignale
mit gleichbleibendem Empfindlichkeitsniveau in Tätigkeit.
Die Anzeigeeinheit ist für die Messung der Echosignaldurchlaufzeit, die mit der zweiten Sollzahl übereinstimmt, und
für die Darstellung der gemessenen Durchlaufzeit in Einheiten der Tiefe des Tierkörpers, die dem reflektierten
Echosignal entspricht, bestimmt.

- 3 -

Ein Nachteil des Messgeräts besteht darin, dass es nicht gestattet, die Dicke der Muskelschicht zu messen und zu reproduzieren sowie die Tierträchtigkeit festzustellen.

Bekannt ist ebenfalls eine Einrichtung zur Trächtigkeitsdiagnostik (US, A, 4 226 229), die ein kompaktes Gehäuse enthält, an dessen Aussenfläche ein Wandler, ein erster Lichtzeiger, der die Berührung des Wandlers mit dem Tierkörper indiziert, ein zweiter Lichtzeiger, der die Trächtigkeit signalisiert, und ein Hörsignalgeber, der dabei ein akustisches Signal abgibt, angeordnet sind. Eine Schaltung, die einen dem Tierkörper Ultraschallimpulse zuführenden Wandler umfasst und die vom Wandler abgefangenen Reflexionsimpulse wahrnimmt, ist ins Gehäuse eingebaut. Die im Laufe des ersten Zeitintervalls wahrgenommenen Reflexionsimpulse werden im ersten Teil der Schaltung analysiert. Er ist mit dem ersten Lichtzeiger verbunden und schaltet ihn im Laufe dieses Intervalls ein, wenn die Echoimpulse bestimmte Eigenschaften besitzen, die durch die Berührung der Sonde mit dem Gewebe bedingt sind. Im zweiten Teil der Schaltung werden die im Laufe des zweiten Zeitintervalls reflektierten Impulse wahrgenommen und ausgewertet. Er ist mit dem zweiten Lichtzeiger und dem Hörsignalgeber verbunden und schaltet diese im Laufe dieses Intervalls ein, wenn die Echosignale bestimmte Eigenschaften besitzen, die durch Vorhandensein des Fruchtwassers in der Gebärmutter des Tieres bedingt sind. Im Gehäuse ist eine Speisequelle der Schaltung angeordnet.

Ein Nachteil dieser Einrichtung besteht darin, dass sie für die Dickenmessung der Fett- und Muskelschicht nicht benutzt werden kann.

Bekannt ist auch ein Ultraschalltester zur Trächtigkeitsfeststellung der Tiere und zur Dickenmessung ihrer Fett- und Muskelschicht (Pig International, V. 16, No. I, January, Scanoprobe II, p. 34). Der Tester ist mit einem Tragriemen und einem über ein Kabel mit dem Tester verbundenen Geber versehen. Der Tester enthält eine Linearskale mit drei Umschaltern zur Messungsdurchführung der Fett- und Muskelschichtdicke sowie zur Trächtigkeitsfeststellung.

- 4 -

Einen Nachteil des Testers bilden seine grossen Aussenabmessungen, das Vorhandensein des Gebers mit Kabel und der Linearskale mit Betriebsartenschaltern, wodurch sein Betrieb erschwert und die Testerleistung vermindert wird.

Bekannt ist ein Fettschichtdickenmesser (US, A, I I55 839) der einen Synchronisator, einen Impulserzeuger, ein Filter, einen Piezoultraschallwandler, dessen Ende über die Grenzen des Gehäuses hinausragt, einen Verstärker, eine Anzeigeeinheit mit zweistelligem Sichtgerät, einen an das Filter angeschlossenen elektronischen Schalter und einen batterie- oder akkumulatorenbetriebenen Speiseteil enthält, die im Gehäuse hintereinandergeschaltet sind.

Der Messer arbeitet wie folgt.

Die Bedienungsperson drückt den Piezowandler des Messers an den Tierkörper. Mit bestimmter Druckkraft erzeugt der Piezowandler solch eine grosse Spannung, dass sie - über das Filter dem Schalter zugeführt - den Speiseteil des Messers anschliesst. Der Synchronisator löst den Impulserzeuger aus, und in der Anzeigeeinheit beginnt die Zeitzählung. Von dem Erzeuger kommt der Impuls über das Filter am Piezowandler an. Der Ultraschallimpuls des Piezowandlers wird von der Grenzfläche zwischen dem Speck und dem Muskelgewebe reflektiert und wieder vom Piezowandler aufgenommen. Der dabei entstehende Impuls geht durch das Filter in den Verstärker und weiter in die Anzeigeeinheit durch. In Ubereinstimmung mit der Laufzeit des Ultraschallimpulses, die von der Speckdicke abhängt, wird ein Messergebnis am Sichtgerät gewonnen. Im nächsten Messzyklus löst der Synchronisator den Erzeuger aus, und der beschriebene Vorgang wiederholt sich.

Ein Nachteil dieses Messers besteht darin, dass die fleischerne Schichtdicke mit seiner Hilfe nicht registriert werden kann, da bei Messungen eine Menge von Echoimpulsen der Ultraschallwellen von den verschieden dichten Geweben erzeugt wird. Zur Dickenmessung der fleischernen Schicht muss der Messer die Echoimpulse von der oberen und unteren Grenze des Rückenmuskels registrieren. Wie bereits erwähnt, wird jedoch keine Trennung der jeweiligen Echoimpulse mit dem Messer ermöglicht. Die Verwendung von Handschaltern ist für die Trennung der jeweiligen Echoimpulse unerwünscht, weil sich die Neigung des Ultraschallstrahles, die Geber- und Tierstellung bei Umschaltungen ändern, was die Genauigkeit des Messergebnisses beeinflusst. Ausserdem ermöglicht der Messer keine Trächtigkeitsfeststellung des Tieres.

Offenbarung der Erfindung

Der Erfindung liegt die Aufgabe zugrunde, ein Ultraschallmessgerät zu entwickeln, dessen Bauart gemeinsame Baugruppen für die Dickenmessung des Fettgewebes und des Rückenmuskels des Schweines auszunutzen sowie dessen Trächtigkeit festzustellen vermag.

Die gestellte Aufgabe wird dadurch gelöst, dass bei dem Echoimpulstester, der einen Synchronisator aufweist, dessen einer Ausgang mit einem der Eingänge einer Anzeigeeinheit und dessen anderer Ausgang mit dem Eingang eines Impulserzeugers verbunden ist, bei dem der Ausgang am Eingang eines Verstärkers und an einem der Eingänge eines Filters liegt, an dessen anderen Eingang ein Ultraschallwandler und an dessen Ausgang ein elektronischer Schalter angeschlossen ist, der mit dem Steuereingang eines Speiseteils in Verbindung steht, dessen Ausgang an den jeweiligen Eingängen des Impulserzeugers, des Synchronisators, des Verstärkers und der Anzeigeeinheit liegt, erfindungsgemäss ein Echoimpulsdekadenzähler und eine Trächtigkeitsdiagnoseschaltung vorgesehen sind, wobei der Ausgang des Verstärkers an die entsprechenden Ausgänge der Trächtigkeitsdiagnoseschaltung und des Echoimpulszählers angeschlossen ist, dessen Ausgänge an den Informationseingängen der Anzeigeeinheit liegen, während der Ausgang der Anzeigeeinheit an den anderen Eingang der Trächtigkeitsdiagnoseschaltung angeschlossen ist, deren Ausgang am anderen Eingang des Verstärkers liegt, und der andere Ausgang des Synchronisators mit einem der Eingänge des Echoimpulszählers verbunden ist, während der Ausgang des Speiseteils an den jeweiligen Eingängen des Echoimpulszählers und der Trächtigkeitsdiagnoseschaltung liegt.

Durch die vorliegende Erfindung wird die Dickenmessung der Fettschicht und des Rückenmuskels des Schweines sowie dessen Trächtigkeitsdiagnostik ermöglicht. Der Dickenmesser ist ein kompaktes, in Kleinbauweise ausgeführtes Gerät. Unter Verwendung von kleinen Batterien als Speisequelle wird es leicht bedienbar und kann ohne das Verbindungskabel, das seinen Betrieb erschwert, verwendet werden. Durch die beanspruchte Bauart des Ultraschalldickenmessers wird sein Betrieb wesentlich vereinfacht.

Im Bedarfsfall kann also der Viehpfleger schnell unmittelbar in der Bucht die Dicke des Fettgewebes, die Dicke des Rückenmuskels des Schweines messen bzw. die Trächtigkeit des Tieres feststellen.

Bei einer bevorzugten Ausführungsform der Erfindung enthält die Trächtigkeitsdiagnoseschaltung untereinander verbundene Zeitverzögerungsformer und ein Hörsignalmittel. Diese Ausführung der Trächtigkeitsdiagnoseschaltung ist in der Bauart höchst einfach und zuverlässig.

Gemäss einer der Ausführungsformen der Erfindung enthält die Anzeigeeinheit ein vierstelliges Sichtgerät, das die Anzeige der Dicke des Fettgewebes und des Rückenmuskels praktisch gleichzeitig ermöglicht.

Kurzbeschreibung der Zeichnungen

Nachstehend werden andere Ziele und Vorteile der vorliegenden Erfindung an Hand eines Ausführungsbeispiels und der beigefügten Zeichnungen näher erläutert; es zeigen:

Fig. I ein prinzipielles Blockschaltbild des erfindungsgemässen Echoimpulstesters;

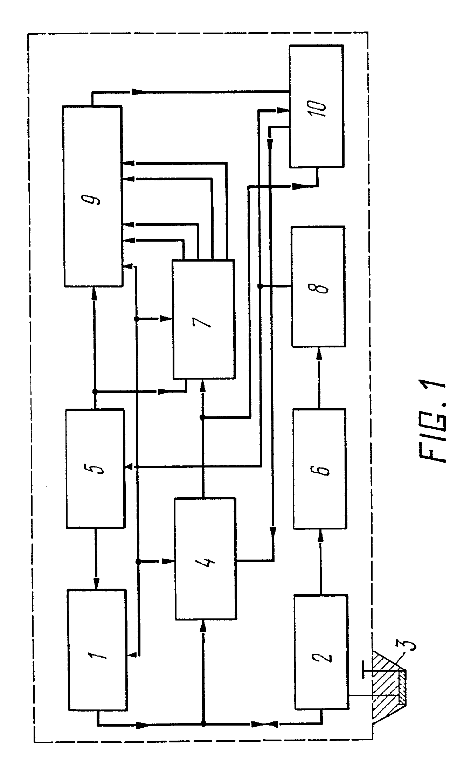

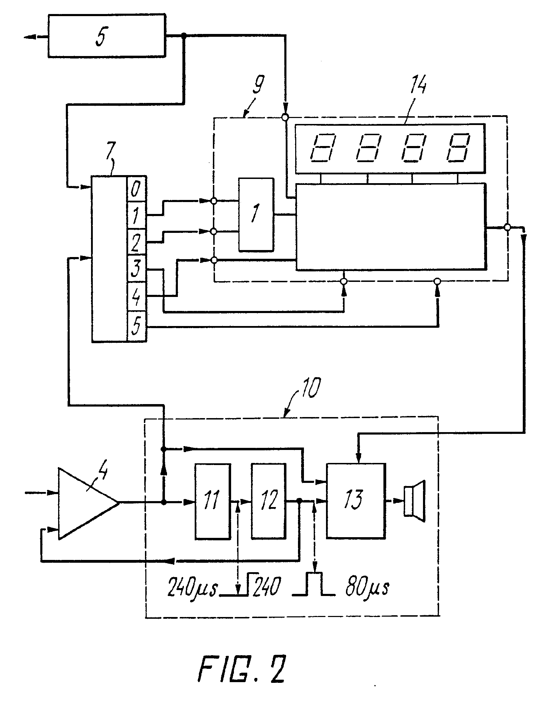

Fig. 2 eine Ausführungsform der Trächtigkeitsdiagnoseschaltung, des Echoimpulszählers und der Anzeigeeinheit;

Fig. 3 einen Gewebeschichtenaufbau des Schweines;

Fig. 4 eine Stellung des Testers bei der Dickenmessung des Fettgewebes und des Rückenmuskels;

Fig. 5 Echoimpulse von verschiedenen Gewebeschichten des Schweines.

Bevorzugte Ausführungsform der Erfindung

Der Echoimpulstester enthält einen über ein Filter 2 mit einem Piezokristall-Ultraschallwandler 3 verbundenen Erzeuger I (Fig. I-2), einen Verstärker 4, dessen Messeingang mit dem Impulserzeuger I und dem Filter 2 verbunden ist, einen Synchronisator 5, einen elektronischen Schalter 6, einen Echoimpulsdekadenzähler 7, einen Speiseteil 8, eine Anzeigeeinheit 9 und eine Trächtigkeitsdiagnoseschaltung IO. Die Schaltung IO enthält Zeitverzögerungsformer II, I2 und ein Hörsignalmittel I3. Die Anzeigeeinheit 9 weist ein vierstelliges Sichtgerät I4 auf.

Der Ausgang des Schalters 6 liegt am Eingang des Speiseteils 8, dessen Ausgang an die entsprechenden Klemmen des Impulserzeugers I, des Verstärkers 4, des Synchronisators 5 und der Anzeigeeinheit 9 sowie an den Zähler 7 und die Schaltung IO angeschlossen ist. Ausserdem liegt der zweite Eingang des Verstärkers 4 am Ausgang der Schaltung IO, während sein erster Eingang an den Ausgang des Impulserzeugers I und den Eingang des Filters 2 angeschlossen ist. Der Ausgang des Verstärkers 4 ist an den zweiten Eingang des Zählers 7 und den zweiten Eingang der Schaltung IO angeschlossen, und die Ausgänge des Synchronisators 5 sind mit dem Eingang des Impulserzeugers I, dem Eingang des Zählers 7 und dem ersten Eingang der Anzeigeeinheit 9 verbunden. Die Ausgänge des Zählers 7 sind an die Informationseingänge des Speiseteils 9 angeschlossen, dessen Ausgang am ersten Eingang der Schaltung IO, d.h. am Hörsignalmittel I3 liegt.

Der Ultraschalldickenmesser arbeitet wie folgt.

I. Dickenmessung der Fettschicht und des Rückenmuskels.

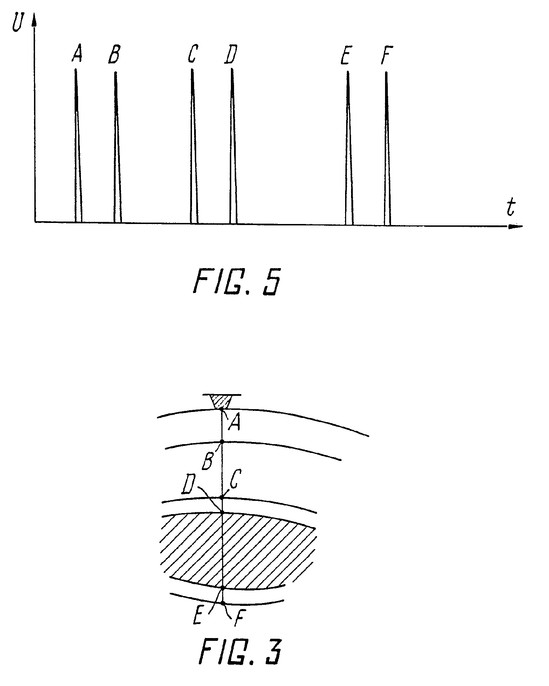

Fig. 3 zeigt einen Rumpfschnitt des Schweines, bei dem die Grenzen der Gewebeschichten mit Punkten A, B, C, D, E, F angedeutet sind.

Vor der Messungsdurchführung wird die Meßstelle (6., 7. Rippe, 2 bis 5 cm von der Wirbelsäule) eingeölt, um die Haarbedeckung an der Haut haften zu lassen und die Berührung des Testers mit dem Tierkörper zu verbessern.

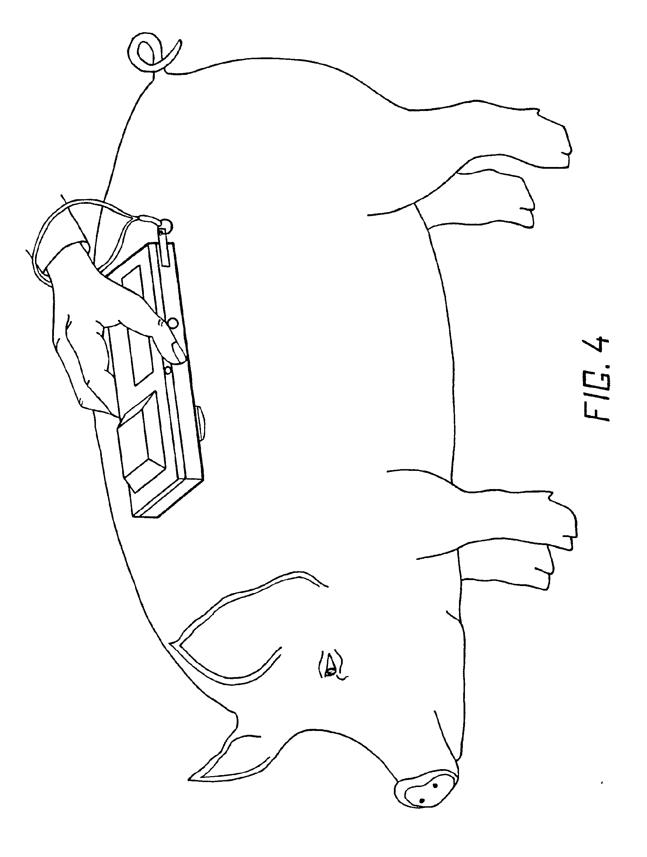

Zunächst wird der Tester in die Hand genommen. Dann drückt man den Ultraschallwandler 3 an die geschmierte Stelle des Tierkörpers (Fig. 4). Mit bestimmter Druckkraft erzeugt der Ultraschallwandler 3 eine so grosse Spannung (2 bis 3 V), dass sie - über das Filter 2 dem hochohmigen Eingang des Schalters 6 zugeführt - den Speiseteil 8 anschliesst. Dabei schalten sich alle Einheiten I, 4, 5, 7, 9, IO ein. Der Synchronisator 5 löst den Erzeuger I aus. Diese Impulse erzeugen im Kristall des Ultraschallwandlers 3 Schwingungen mit einer Frequenz von 2,5 MHz (in Form eines kurzen Impulspaketes), die durch die Rumpfschicht durchlaufen und von den entsprechenden Schichten reflektiert, von demselben Kristall des Wandlers 3 empfangen und über das Filter 2 dem Eingang des Verstärkers 4 zugeführt werden. Das Filter 2 dient zur Trennung der Echoimpulse und eines Gleichstromsignals (2 bis 3 V), das beim Druck des Wandlers gegen den Tierkörper erscheint. Vom Eingang des Verstärkers 4 fallen die Echoimpulse in den Zähler 7 ein, an dessen ersten Eingang sich die Spannung im Laufe der Zeit hält, die dem Echoimpulsabstand A-C entspricht (Fig. 5). Mit der Ankunft des Echoimpulses C setzt eine Spannung am zweiten Ausgang des Zählers 7 ein (am ersten Ausgang wird die Spannung gleich Null) und leuchtet in der Anzeigeeinheit 9 die Dickengrösse der Fettschicht in mm aus (zwei erste Ziffern des Sichtgerätes I4). Die Zeit zwischen den Reflexionen D-E stimmt mit der Echoimpulswirkzeit am dritten Ausgang des Zählers 7 überein. Mit dem Spannungseinsatz am vierten Ausgang des Zählers 7 wird die Dickengrösse des Rückenmuskels in mm ausgeleuchtet (zwei nächste Ziffern des Sichtgerätes I4). Die Ergebnisse bleiben also in der Sichtgerät-Anzeige.        Einige Sekunden (z. B. I bis 5 s) später wird der Tester ausgeschaltet. Durch einen nochmaligen Druck des Ultraschallwandlers 3 gegen den Tierkörper kann der Messvorgang wiederholt werden.

2. Trächtigkeitsfeststellung

Bei der Trächtigkeitsfeststellung ist der Ultraschallwandler 3   4 bis 6 cm vor dem Hinterbein des Tieres und 2 bis 4 cm oberhalb der letzten Zitze angeordnet. Ein Echosignal von der Gebärmutter wird im Ultraschallwandler 3 empfangen und über das Filter 2 dem Verstärker 4 zugeführt. Da das Echosignal bei der Trächtigkeitsfeststellung aus einer Tiefe von etwa I8 bis 24 cm ankommt, ist es viel schwächer als das Signal für die Dicke des Fettgewebes und des Rückenmuskels. Im Verstärker 4 wird daher durch ein Zeitsignal von der Schaltung 10 eine automatische Verstärkungsänderung vorgenommen. Im Laufe der ersten 240 Mikrosekunden wird die Verstärkung nach der Zuführung eines Sondierimpulses mit einem Ausgangssignal der Schaltung 10 vermindert. 240 bis 320 $\mu$s lang bleibt das Signal am Ausgang der Schaltung 10 aus, und die Verstärkung des Verstärkers 4 wird maximal. Wenn ein Echosignal von der Gebärmutter im Laufe dieser Zeitspanne erscheint, so schaltet es in der Schaltung 10 nach der Verstärkung im Verstärker 4 das Hörsignalmittel 13 ein, an dessen Ausgang ein Hörsignal mit der Frequenz des von dem Ausgang der Anzeigeeinheit 9 ankommenden Signals erscheint.

## Gewerbliche Anwendbarkeit

Die vorliegende Erfindung kann in der Tierzucht, hauptsächlich in der Schweinezucht zur Selektion der Schweine nach Erbmerkmalen (Fettschicht- und Rückenmuskeldicke) und zur Kontrolle der Frühgravidität (Trächtigkeit) nach ihrer Paarung oder künstlichen Insemination angewendet werden.

PATENTANSPRÜCHE

I. Echoimpulstester, der einen Synchronisator (5) aufweist, dessen einer Ausgang mit einem der Eingänge einer Anzeigeeinheit (9) und dessen zweiter Ausgang mit dem Eingang eines Impulserzeugers (I) verbunden ist, dessen Ausgang am Eingang eines Verstärkers (4) und an einem der Eingänge eines Filters (2) liegt, an dessen anderen Eingang ein Ultraschallwandler (3) und an dessen Ausgang ein elektronischer Schalter (6) angeschlossen ist, der mit dem Steuereingang eines Speiseteils (8) in Verbindung steht, dessen Ausgang an den jeweiligen Eingängen des Impulserzeugers (I), des Synchronisators (5), des Verstärkers (4) und der Anzeigeeinheit (9) liegt, dadurch g e k e n n z e i c h n e t, dass er mit einem Echoimpulsdekadenzähler (7) und einer Trächtigkeitsdiagnoseschaltung (IO) versehen ist, wobei der Ausgang des Verstärkers (4) an die entsprechenden Eingänge der Trächtigkeitsdiagnoseschaltung (IO) und des Echoimpulszählers (7) angeschlossen ist, dessen Ausgänge an den Informationseingängen der Anzeigeeinheit (9) liegen, während der Ausgang der Anzeigeeinheit (9) an den anderen Eingang der Trächtigkeitsdiagnoseschaltung (IO) angeschlossen ist, deren Ausgang am anderen Eingang des Verstärkers (4) liegt, und der andere Ausgang des Synchronisators (5) mit einem der Eingänge des Echoimpulszählers (7) ist verbunden,, während der Ausgang des Speiseteils (8) an den jeweiligen Eingängen des Echoimpulszählers (7) und der Trächtigkeitsdiagnoseschaltung (IO) liegt.

2. Echoimpulstester nach Anspruch I, dadurch g e - k e n n z e i c h n e t, dass die Trächtigkeitsdiagnoseschaltung (IO) Zeitverzögerungsformer (II, I2) und ein Hörsignalmittel (I3) enthält.

3. Echoimpulstester nach Anspruch I, dadurch g e - k e n n z e i c h n e t, dass die Anzeigeeinheit (9) ein vierstelliges Sichtgerät (I4) enthält.

FIG.1

FIG. 2

FIG. 5

FIG. 3

FIG. 4

### I. CLASSIFICATION OF SUBJECT MATTER (if several classification symbols apply, indicate all) 6

According to International Patent Classification (IPC) or to both National Classification and IPC

Int.Cl.$^4$ A61B 8/08, G01B 17/02

### II. FIELDS SEARCHED

**Minimum Documentation Searched 7**

| Classification System | Classification Symbols |
|---|---|
| Int.Cl.$^4$ | A61B 8/08,10/00;G01B 17/02;G01S 15/18,15/88; G01N 29/00 |

**Documentation Searched other than Minimum Documentation to the Extent that such Documents are Included in the Fields Searched 8**

### III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | US, A, 4226229 (Thomas D. Eckhart et al.) 7 October 1980 (07.10.80) see the claims,figure 3 | 1-3 |
| A | DE, C2, 2926151 (Tokyo Shibaura Denki K.K.) 18 March 1982 (18.03.82) see the claims, figures 2,3 | 1-3 |
| A | DE, A1, 3211719 (Yokogawa Electric Works, Ltd) 15 September 1983 (15.09.83) see the claims,figures 5,6 | 1-3 |
| A | JP, B2, 5740772 , 30 August 1982 (30.08.82) see figure 1 | 1-3 |
| A | JP, B2, 57-40773, 30 August 1982 (30.08.82) see figures 1-4 | 1-3 |
| A | US, A, 4398540 (Tokyo Shibaura Denki Kabushiki Kaisha) 16 August 1983 (16.08.83) see the claims,figures 3,11 | 1-3 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

### IV. CERTIFICATION

| Date of the Actual Completion of the International Search | Date of Mailing of this International Search Report |
|---|---|
| 18 May 1988 (18.05.88) | 8 August 1988 (08.08.88) |

| International Searching Authority | Signature of Authorized Officer |
|---|---|
| ISA/SU | |

Form PCT/ISA/210 (second sheet) (January 1985)